# EUROPÄISCHE PATENTSCHRIFT

(11) **EP 4 256 026 B1**
(45) Veröffentlichungstag und Bekanntmachung des Hinweises auf die Patenterteilung: **03.07.2024**
(21) Anmeldenummer: 22731507.4
(22) Anmeldetag: 23.05.2022
(51) Int. Cl.: C12M 1/00, B01D 29/35, B01D 45/14, B04B 1/04, B04B 11/02, B07B 7/083

(54) **ROTATIONSABSCHEIDER ZUM ABSCHEIDEN VON FREMDKÖRPERN AUS EINER MEDIENSTRÖMUNG**
ROTARY SEPARATOR FOR SEPARATING FOREIGN BODIES FROM A MEDIA FLOW
SÉPARATEUR ROTATIF POUR SÉPARER DES CORPS ÉTRANGERS D'UN FLUX DE MILIEUX

(30) Priorität: 24.02.2022 DE 102022104496
(43) Veröffentlichungstag der Anmeldung: 11.10.2023
(73) Patentinhaber: Börger GmbH, 46325 Borken-Weseke (DE)
(72) Erfinder: BÖRGER, Alois, 46325 Borken-Weseke (DE); GARVERT, Jan Bernd, 46414 Rhede (DE)
(74) Vertreter: Pelster Behrends Patentanwälte PartG mbB
(86) Internationale Anmeldenummer: PCT/EP2022/063936
(87) Internationale Veröffentlichungsnummer: WO 2023/160833

(56) Entgegenhaltungen:
- EP-A1- 0 004 145
- EP-A1- 1 972 691
- WO-A2-2019/185650
- DE-A1- 10 163 924
- DE-A1-102006 052 669
- DE-A1-102016 214 914
- DE-T2- 69 104 081
- US-A- 4 334 647
- US-A1- 2009 211 439
- US-A1- 2015 218 506
- US-A1- 2015 298 142
- US-A1- 2017 162 594
- US-A1- 2020 230 615

## Beschreibung

Die Erfindung betrifft einen Rotationsabscheider zum Abscheiden von Fremdkörpern aus einer Medienströmung, insbesondere aus einer Biogassubstratströmung, mit einem Abscheidergehäuse, welches einen Abscheidebereich für eine Fremdkörperabscheidung aus der Medienströmung und einen Auslassbereich zum Ausleiten der Medienströmung nach erfolgter Fremdkörperabscheidung aufweist, und einen zumindest abschnittsweise in dem Abscheidebereich des Abscheidergehäuses angeordneten und rotierend antreibbaren Rotor, wobei der Rotor zumindest eine Abscheideschaufel umfasst und der Abscheidebereich über einen Verbindungskanal mit dem Auslassbereich verbunden ist, dadurch gekennzeichnet, dass der Verbindungskanal ausserhalb des Rotors ausgebildet ist.

Ferner betrifft die Erfindung ein Abscheidesystem mit einer Fördereinheit zum Erzeugen oder Unterstützen einer Medienströmung, insbesondere einer Biogassubstratströmung, innerhalb des Abscheidesystems, und einem von der Fördereinheit beabstandeten Rotationsabscheider zum Abscheiden von Fremdkörpern aus der Medienströmung.

Darüber hinaus betrifft die Erfindung ein Verfahren zum Abscheiden von Fremdkörpern aus einer Medienströmung, insbesondere aus einer Biogassubstratströmung, mittels eines Rotationsabscheiders, mit den Schritten: Einleiten der Medienströmung in einen Abscheidebereich eines Abscheidergehäuses des Rotationsabscheiders, Abscheiden von Fremdkörpern aus der Medienströmung mittels eines zumindest abschnittsweise in dem Abscheidebereich des Abscheidergehäuses angeordneten und rotierend angetriebenen Rotors des Rotationsabscheiders und Ausleiten der Medienströmung nach erfolgter Fremdkörperabscheidung aus einem Auslassbereich des Abscheidergehäuses des Rotationsabscheiders.

Aus der Druckschrift DE 691 04 081 T2 ist ein zentrifugal wirkender Windsichter bekannt. Aus der Druckschrift EP 0 004 145 A1 ist ein Vakuumstaubsauger bekannt. Aus der Druckschrift DE 10 2006 052 669 A1 ist ein Verfahren zum Eindicken von Gärsubstrat aus einer Biogasanlage bekannt. Aus der Druckschrift EP 1 972 691 A1 ist ein Verfahren zur Herstellung von Biogas durch diskontinuierliche, 2-stufige Trockenfermentation bekannt. Aus der Druckschrift US 2009 / 0 211 439 A1 ist ein Abscheider zur Abscheidung von Partikeln aus einem Gasstrom bekannt. Aus der Druckschrift DE 101 63 924 A1 ist ein Verfahren zum Separieren einer partikel- oder tröpfchenförmigen Verunreinigung aus einem Trägerfluid bekannt.

In einer Vielzahl von Anwendungsfällen ist es erforderlich oder zumindest vorteilhaft, Fremdkörper aus einer Medienströmung abzuscheiden. In Bezug auf Biogasanlagen ist es beispielsweise erforderlich, Fremdkörper, wie beispielsweise Steine, aus einer Biogassubstratströmung abzuscheiden, bevor die Biogassubstratströmung einem Fermenter zugeführt wird, damit die Biogasanlage nicht durch die Fremdkörper beschädigt wird und eine Akkumulation von Fremdkörpern innerhalb der Biogasanlage vermieden wird. Auch in anderen Bereichen müssen Feststoffe aus Medienströmungen abgeschieden werden, damit nachfolgende Prozesse, in denen beispielsweise fremdkörperempfindliche Verdrängerpumpen eingesetzt werden, nicht durch die Feststoffe beschädigt werden.

Im Stand der Technik sind bisher keine praxistauglichen Abscheideeinrichtungen bekannt, mittels welchen Fremdkörper in einer Biogassubstratströmung zuverlässig abscheidbar sind.

Die der Erfindung zugrundeliegende Aufgabe besteht somit darin, die Abscheidung von Fremdkörpers aus einer Medienströmung, insbesondere einer Biogassubstratströmung, zu ermöglichen oder zumindest zu vereinfachen.

Die Aufgabe wird gelöst durch einen Rotationsabscheider gemäß Anspruch 1, dem Abscheidesystem gemäß Anspruch 14 und dem Verfahren gemäß Anspruch 15.

Der Abscheidebereich befindet sich auf der Rohseite des Rotationsabscheiders. Der Auslassbereich befindet sich auf der Reinseite des Rotationsabscheiders. Beispielsweise kann durch eine in Strömungsrichtung hinter dem Verbindungskanal angeordnete Fördereinrichtung über den Verbindungskanal eine Saugwirkung in dem Abscheidebereich erzeugt werden. Es entsteht eine sich vor dem Verbindungskanal befindende Ansaugzone, durch welche das Medium von dem Abscheidebereich in den Auslassbereich gesaugt wird. Durch die rotatorische Bewegung des Rotors werden die sich innerhalb des Abscheidebereichs befindenden Fremdkörper radial nach außen an die den Abscheidebereich umgebende Innenwand des Abscheidergehäuses gefördert, sodass verhindert wird, dass die Fremdkörper durch den Verbindungskanal in den Auslassbereich innerhalb des Abscheidergehäuses gelangen. Der Rotationsabscheider erlaubt also eine kontinuierliche Fremdkörperabscheidung aus der Medienströmung, sodass der Rotationsabscheider zum Zwecke der Fremdkörperabscheidung ohne Beeinträchtigung der Medienströmung in ein Strömungssystem integriert werden kann.

Die abzuscheidenden Fremdkörper sind vorzugsweise Feststoffe. Die Feststoffe sind vorzugsweise Schwerteile, welche eine höhere Dichte als die übrigen Bestandteile der Medienströmung aufweisen.

Der Rotor umfasst eine oder mehrere Abscheideschaufeln Die eine oder die mehreren Abscheideschaufeln sind vorzugsweise im Abscheidebereich angeordnet. Die eine oder die mehreren Abscheideschaufeln scheiden die Fremdkörper nicht unmittelbar aus der Medienströmung ab, verursachen im Abscheidebereich jedoch die Ausbildung einer Strömung, welche zum Abscheiden der Fremdkörper genutzt wird. Die eine oder die mehreren Abscheideschaufeln sind vorzugsweise gekrümmt ausgebildet. Bei mehreren Abscheideschaufeln sind diese vorzugsweise gleichmäßig beabstandet auf einer Kreisbahn um den Verbindungskanal angeordnet. Die in den Abscheidebereich ragenden Außenkanten der Abscheideschaufeln können auf einer konisch zulaufenden Fläche liegen und/oder nach innen gebogen bzw. gewölbt sein. Die Abscheideschaufeln können auch axial beabstandet von dem Verbindungskanal angeordnet sein.

Das Abscheidergehäuse des Rotationsabscheiders umfasst vorzugsweise einen Gehäusedeckel, welcher insbesondere werkzeuglos und/oder zerstörungsfrei und reversibel lösbar an einem Basiskörper des Abscheidergehäuses befestigt ist. Durch den Gehäusedeckel ist vorzugsweise ein direkter Zugang zum Rotor innerhalb des Abscheidergehäuses möglich.

Der Rotationsabscheider kann eine Antriebseinrichtung aufweisen, über welche der Rotor rotatorisch angetrieben wird. Die Antriebseinrichtung kann z.B. eine elektrische, pneumatische oder hydraulische Antriebseinrichtung sein.

Die Medienströmung kann eine Strömung einer Suspension mit beigemengten Störstoffen, wie etwa Sand, Steinen, Glas und/oder Metall, sein. Die Medienströmung kann ferner eine Gülleströmung, eine Sand-Wasser-Kieselgemisch-Strömung oder eine Strömung einer Entsorgungsflüssigkeit sein, welche Feststoffe beinhaltet, etwa zu entsorgende zerkleinerte Essensreste, denen Flaschen- und Besteckteile beigemischt sein können.

Der Verbindungskanal des Rotationsabscheiders ist außerhalb des Rotors ausgebildet. Vorzugsweise wird der Verbindungskanal durch eine oder mehrere von dem Rotor beabstandete Strömungsdurchlässe zwischen dem Abscheidebereich und dem Auslassbereich gebildet. Der eine oder die mehreren Strömungsdurchlässe können in dem Abscheidergehäuse angeordnete Rohroder Leitungssegmente und/oder Ausnehmungen oder Öffnungen in einem in dem Abscheidergehäuse angeordneten Trennkörper, beispielsweise einem Trennblech, zwischen dem Abscheidebereich und dem Auslassbereich sein. Der Verbindungskanal kann ein umlaufender, beispielsweise ringförmiger, Strömungsdurchlass sein, welcher um den Rotor umläuft.

In einer bevorzugten Ausführungsform des erfindungsgemäßen Rotationsabscheiders ist die zumindest eine Abscheideschaufel als Bestandteil eines, insbesondere um den Verbindungskanal umlaufenden, Abscheideschaufelkranzes ausgebildet. Der Abscheideschaufelkranz kann auch axial beabstandet von dem Verbindungskanal angeordnet sein. Der Abscheideschaufelkranz umfasst mehrere Abscheideschaufeln. Beispielsweise umfasst der Abscheideschaufelkranz zwei, drei, vier, fünf oder mehr als fünf Abscheideschaufeln. Die Abscheideschaufeln sind beispielsweise an einem den Verbindungskanal bildenden Rohrsegment des Rotors befestigt. Der Rotor oder der Abscheidekranz sind vorzugweise aus Metall ausgebildet.

In einer anderen bevorzugten Ausführungsform des erfindungsgemäßen Rotationsabscheiders verläuft der Verbindungskanal koaxial zu der Rotationsachse des Rotors. Der Verbindungskanal wird vorzugsweise durch ein zentrales Hohlrohr des Rotors gebildet. Die fremdkörperfreie Strömung bildet sich insbesondere im Nahbereich der Rotationsachse des Rotors aus. Durch die koaxiale Anordnung des Verbindungskanals zu der Rotationsachse des Rotors wird die Medienströmung von dem Abscheidebereich in den Auslassbereich unterstützt.

In einer anderen bevorzugten Ausführungsform des erfindungsgemäßen Rotationsabscheiders wird der Verbindungskanal durch eine oder mehrere Ausnehmungen in einem Trennkörper zwischen dem Abscheidebereich und dem Auslassbereich gebildet wird. Der Trennkörper kann ein Trennblech sein. Der Trennkörper kann eine feststehende Gegenschneide für einen oder mehrere Schneidkörper einer Schneideinrichtung bilden.

Es ist darüber hinaus ein erfindungsgemäßer Rotationsabscheider vorteilhaft, bei welchem die zumindest eine Abscheideschaufel dazu eingerichtet ist, durch eine Rotationsbewegung eine Strömung, insbesondere eine Wirbelströmung, innerhalb des Abscheidebereichs des Abscheidergehäuses zu verursachen, mittels welcher Fremdkörper durch Fliehkrafteinwirkung radial nach außen gefördert werden. Dadurch, dass die Fremdkörper durch Fliehkrafteinwirkung radial nach außen gefördert werden, wird verhindert, dass die Fremdkörper durch den Verbindungskanal in den Auslassbereich strömen. Durch die anhaltendende Wirbelströmung werden die radial nach außen geförderten Fremdkörper an der Innenwand des Abscheidergehäuses gehalten, sodass ein äußerst geringes Risiko des Eintritts eines Fremdkörpers in den Verbindungskanal besteht.

In einer anderen bevorzugten Ausführungsform des erfindungsgemäßen Rotationsabscheiders befindet sich zwischen dem Abscheidebereich und dem Auslassbereich ein Trennkörper, welcher dazu eingerichtet ist, eine Medienströmung zwischen dem Abscheidebereich und dem Auslassbereich jenseits des Verbindungskanals im Wesentlichen zu verhindern. Der Trennkörper kann eine Platte sein. Der Trennkörper kann feststehend ausgebildet sein. Der Trennkörper kann an dem Abscheidergehäuse fixiert sein oder als Bestandteil des Abscheidergehäuses ausgebildet sein. Alternativ kann der Trennkörper dazu eingerichtet sein, sich mit dem Rotor mitzudrehen. Ferner kann der Trennkörper Bestandteil des Rotors sein.

Der erfindungsgemäße Rotationsabscheider wird ferner durch einen Fremdkörperauslass vorteilhaft weitergebildet, über welchen Fremdkörper aus dem Abscheidebereich oder dem Abscheidergehäuse ausleitbar sind. Vorzugsweise umfasst der Rotationsabscheider ein oder mehrere Leitelemente, welche dazu eingerichtet sind, Fremdkörper in einer durch die zumindest eine Abscheideschaufel verursachten Strömung in Richtung des Fremdkörperauslasses zu leiten. Das eine oder die mehreren Leitelemente können Leitstege sein. Das eine oder die mehreren Leitelemente können Bestandteile des Abscheidergehäuses sein und/oder an der Innenwand des Abscheidergehäuses angeordnet sein. Das eine oder die mehreren Leitelemente können stationäre bzw. feststehende sich an der Innenwand des Abscheidergehäuses befindende Schneckenflügel sein. Bei einer Medienströmung, in welcher die Fremdkörper Steine sind, kann der Fremdkörperauslass auch als Steinfang bezeichnet werden. An den Fremdkörperauslass kann sich eine Fremdkörperauslassleitung anschließen, welche sich in tangentialer Richtung in Bezug auf den Abscheidebereich erstreckt. Der Fremdkörperauslass kann mit einer Entnahmeschleuse des Rotationsabscheiders verbunden sein, welcher im laufenden Betrieb des Rotationsabscheiders entleert werden kann. Bei einer Entleerung der Entnahmeschleuse ergibt sich lediglich ein minimaler Verlust an Flüssigkeit.

In einer anderen bevorzugten Ausführungsform weist der erfindungsgemäße Rotationsabscheider einen Medieneinlass auf, über welchen die Medienströmung in den Abscheidebereich einleitbar ist, wobei der Medieneinlass vorzugweise an einer dem Rotor gegenüberliegenden Stirnseite des Abscheidebereichs angeordnet ist. Alternativ ist der Medieneinlass am Außenumfang des Abscheidebereichs angeordnet. Wenn der Medieneinlass am Außenumfang des Abscheidebereichs angeordnet ist, ergibt sich vorzugsweise ein schräger oder ein gerader Eintrittswinkel für die Medienströmung, sodass die abzuscheidenden Fremdkörper in Richtung des Fremdkörperauslasses gelenkt werden. Die Einlassleitung, welche den Eintrittswinkel der Medienströmung vorgibt, kann einen Schrägstellungswinkel in Bezug auf die Querachse des Abscheidergehäuses des Rotationsabscheiders aufweisen. Der Schrägstellungswinkel der Einlassleitung und/oder der Eintrittswinkel der Medienströmung können in einem Bereich zwischen 5 Grad und 45 Grad, vorzugsweise in einem Bereich zwischen 10 Grad und 30 Grad, liegen.

Der Medieneinlass am Außenumfang des Abscheidebereichs kann mit einer Einlassleitung verbunden sein, welche sich in tangentialer Richtung in Bezug auf den Abscheidebereich erstreckt. Vorzugsweise weist der Rotationsabscheider einen Medienauslass auf, über welchen die Medienströmung nach erfolgter Fremdkörperabscheidung aus dem Abscheidergehäuse ausleitbar ist, wobei der Medienauslass vorzugweise am Außenumfang des Abscheidebereichs angeordnet ist. Der Medienauslass am Außenumfang des Abscheidebereichs kann mit einer Auslassleitung verbunden sein, welche sich in tangentialer Richtung in Bezug auf den Auslassbereich erstreckt. Wenn die Auslassleitung in einem oberen Bereich des Auslassbereichs angeordnet ist, kann Luft oder Gas das Abscheidergehäuse ohne weiteres verlassen.

Es ist darüber hinaus ein erfindungsgemäßer Rotationsabscheider mit einer rotierend antreibbaren Fördereinrichtung bevorzugt. Die Fördereinrichtung wirkt als Eigenpumpe des Rotationsabscheiders. Die rotierend antreibbare Fördereinrichtung ist in dem Abscheidergehäuse des Rotationsabscheiders angeordnet und weist vorzugsweise einen oder mehrere Förderschaufeln auf. Die Fördereinrichtung ist vorzugsweise in dem Auslassbereich in dem Abscheidergehäuse angeordnet. Mittels der Fördereinrichtung kann eine Strömung von dem Abscheidebereich durch den Verbindungskanal in den Auslassbereich erzeugt oder unterstützt werden. Durch die Fördereinrichtung wird der Flusswiederstand des Rotationsabscheiders in einem Abscheidesystem überkompensiert, sodass der Rotationsabscheider auf der Saugseite einer Fördereinheit, beispielsweise einer Drehkolbenpumpe, des Abscheidesystems platziert werden kann und die nachgeschaltete Fördereinheit somit vor Kavitation schützen.

In einer Weiterbildung des erfindungsgemäßen Rotationsabscheiders ist die Fördereinrichtung als Bestandteil des Rotors ausgebildet oder drehsteif mit dem Rotor verbunden. Die eine oder die mehreren Abscheideschaufeln und die eine oder die mehreren Förderschaufeln werden vorzugsweise über dieselbe Antriebswelle und/oder mittels derselben Antriebseinrichtung angetrieben. Der Rotor kann demnach ein Abscheide- und Förderrotor sein. Der Rotor ist in diesem Fall abschnittsweise in dem Abscheidebereich und abschnittsweise in dem Auslassbereich innerhalb des Abscheidergehäuses des Rotationsabscheiders angeordnet.

Der erfindungsgemäße Rotationsabscheider wird ferner dadurch vorteilhaft weitergebildet, dass die Fördereinrichtung als Zentrifugalpumpe ausgebildet ist. Die Fördereinrichtung ist vorzugsweise als Hohlwellenzentrifugalpumpe ausgebildet, wobei die Hohlwelle der Zentrifugalpumpe den den Abscheidebereich mit dem Auslassbereich verbindenden Verbindungskanal bildet.

Es ist darüber hinaus ein erfindungsgemäßer Rotationsabscheider bevorzugt, bei welchem sich der Verbindungskanal in die Fördereinrichtung erstreckt und eine oder mehrere Durchtrittsöffnungen in der Kanalbewandung aufweist, über welche die Medienströmung der einen oder den mehreren Förderschaufeln der Fördereinrichtung zuleitbar ist. Das Medium strömt innerhalb des Verbindungskanals im Wesentlichen in axialer Richtung. Durch die eine oder die mehreren Durchtrittsöffnungen kann die Medienströmung in Radialrichtung aus dem Verbindungskanal austreten und über eine Auslassöffnung aus dem Rotationsabscheider ausgeleitet werden.

In einer anderen Ausführungsform des erfindungsgemäßen Rotationsabscheiders ist der Verbindungskanal feststehend in dem Abscheidergehäuse angeordnet. Vorzugsweise erstreckt sich der Rotor durch den Verbindungskanal. Der Verbindungskanal ist also separat von dem Rotor ausgebildet, sodass eine Drehbewegung des Rotors nicht auf den Verbindungskanal übertragen wird. Der Verbindungkanal schließt sich vorzugsweise an eine Durchströmungsöffnung in einer Trennplatte zwischen dem Abscheidebereich und dem Auslassbereich an. Der Verbindungskanal kann an der Trennplatte befestigt sein.

In einer Weiterbildung des erfindungsgemäßen Rotationsabscheiders ist der Rotor in einem Abschnitt als Förderschnecke ausgebildet. Der als Förderschnecke ausgebildete Abschnitt des Rotors kann durch den Verbindungkanal verlaufen oder außerhalb des Verbindungskanals angeordnet sein. Über die Förderschnecke wird ein Schneckeneinzug umgesetzt. Die Förderschnecke unterstützt die Förderung der Medienströmung durch das Abscheidergehäuse, beispielsweise durch den Verbindungskanal. Insbesondere bei Medienströmungen mit langfaserigen Bestandteilen verhindert die Förderschnecke eine Verstopfung des Verbindungskanals oder reduziert zumindest die Verstopfungsanfälligkeit. Ferner können auftretende faserbedingte Verstopfungen des Verbindungkanals mittels der Förderschnecke effektiv aufgelöst werden, da die Förderschnecke Faserbestandteile durch den Verbindungkanal durchzieht. Vorzugsweise umfasst der Rotor einen als Förderschnecke ausgebildeten Abschnitt und zusätzlich eine Abscheideeinrichtung mit einer oder mehreren Abscheideschaufeln. Der als Förderschnecke ausgebildete Abschnitt des Rotors ist vorzugsweise auf der dem Medieneinlass zugewandten Seite der einen oder der mehreren Abscheideschaufeln angeordnet und dazu eingerichtet, die durch den Medieneinlass einströmende und Fremdkörper beinhaltende Medienströmung in Richtung der einen oder der mehreren Abscheideschaufeln zu fördern.

In einer weiteren Ausführungsform des erfindungsgemäßen Rotationsabscheiders weist der den als Förderschnecke ausgebildeten Abschnitt des Rotors umgebende Teilbereich des Abscheidergehäuses einen kleineren Querschnitt, insbesondere einen kleineren Durchmesser, auf als der die eine oder die mehreren Abscheideschaufeln umgebende Teilabschnitt des Abscheidergehäuses. Wenn der Rotor einen Abscheideschaufelkranz mit mehreren Abscheideschaufeln umfasst, weist der den als Förderschnecke ausgebildeten Abschnitt des Rotors umgebende Teilbereich des Abscheidergehäuses einen kleineren Querschnitt, insbesondere einen kleineren Durchmesser, auf als der Abscheideschaufelkranz. Der als Förderschnecke ausgebildete Abschnitt des Rotors kann abschnittsweise oder vollständig in einer rohrförmigen Einlassleitung des Abscheidergehäuses angeordnet sein.

Die Einlassleitung kann einen Aufweitungsabschnitt aufweisen, in welchem sich der Querschnitt, insbesondere der Durchmesser, der Einlassleitung in Strömungsrichtung der Medienströmung vergrößert. Über einen Flansch kann die Einlassleitung mit einer Saugleitung verbunden werden, über welche der Rotationsabscheider beschickt wird.

Die Auslassleitung kann mit einer Druckleitung verbunden werden, über welche die Medienströmung nach erfolgter Fremdkörperabscheidung im Rotationsabscheider außerhalb des Rotationsabscheiders geleitet wird. Der erfindungsgemäße Rotationsabscheider wird ferner durch eine Schneideinrichtung vorteilhaft weitergebildet. Die Schneideinrichtung ist dazu eingerichtet, Fasermaterial der Medienströmung vor dem Eintritt in den Verbindungskanal oder innerhalb des Verbindungskanals und/oder vor dem Einströmen in den Auslassbereich zu zerschneiden. Durch die Schneideinrichtung können Verstopfungen des Verbindungskanals zwischen Abscheidebereich und Auslassbereich aufgrund von langem Fasermaterial, beispielsweise Stroh, in der Medienströmung verhindert werden. Die Schneideinrichtung kann einen oder mehrerer Schneidkörper, beispielsweise Messer oder Klingen, aufweisen, welche in Strömungsrichtung der Medienströmung vor dem Verbindungskanal oder innerhalb des Verbindungskanals oder vor dem Auslassbereich angeordnet sind. Ein oder mehrere Schneidkörper können feststehend ausgebildet sein. Der eine oder die mehreren feststehenden Schneidkörper sind vorzugsweise an dem Abscheidergehäuse befestigt und/oder in dem Abscheidebereich angeordnet. Ein oder mehrere Schneidkörper können als rotierende Schneidkörper ausgebildet sein. Der eine oder die mehreren rotierenden Schneidkörper können Bestandteile des Rotors sein. Ein oder mehrere rotierende Schneidkörper können fliegend gelagert sein. Beispielsweise sind ein oder mehrere Schneidkörper an der Einströmöffnung des Verbindungkanals oder unmittelbar vor dem Verbindungskanal angeordnet.

In einer anderen bevorzugten Ausführungsform des erfindungsgemäßen Rotationsabscheiders weist die Schneideinrichtung einen oder mehrere an dem Rotor angeordnete Schneidkörper auf. Die Schneidkörper sind vorzugsweise Messer oder Klingen. Die Schneidkörper können starr an dem Rotor befestigt sein oder fliegend an dem Rotor gelagert sein, sodass sich beispielsweise eine fliegende Messeranordnung ergibt. Die fliegende Lagerung der Schneidkörper erlaubt vorzugsweise ein Kipp- oder Schwenkbewegung der Schneidkörper. Durch die fliegende Lagerung können die Schneidkörper Ausweichbewegungen ausführen, wodurch bei einem Fremdkörperkontakt der Schneidkörper Beschädigungen am Rotor vermieden werden.

Die der Erfindung zugrundeliegende Aufgabe wird ferner durch ein Abscheidesystem der eingangs genannten Art gelöst, wobei der Rotationsabscheider des erfindungsgemäßen Abscheidesystems nach einer der vorstehend beschriebenen Ausführungsformen ausgebildet ist. Hinsichtlich der Vorteile und Modifikationen des erfindungsgemäßen Abscheidesystems wird somit zunächst auf die Vorteile und Modifikationen des erfindungsgemäßen Rotationsabscheiders verwiesen. Die Fördereinheit ist in diesem Fall separat vom Rotationsabscheider ausgebildet.

In einer bevorzugten Ausführungsform des erfindungsgemäßen Abscheidesystems ist die Fördereinheit als Verdrängerpumpe, insbesondere als Drehkolbenpumpe, ausgebildet. Alternativ oder zusätzlich ist die Fördereinheit in Strömungsrichtung der Medienströmung vor oder hinter dem Rotationsabscheider angeordnet. Wenn die Fördereinheit in Strömungsrichtung vor dem Rotationsabscheider angeordnet ist, verursacht die Fördereinheit einen Förderdruck, über welchen das Medium durch den Rotationsabscheider gefördert wird. Wenn die Fördereinheit in Strömungsrichtung hinter dem Rotationsabscheider angeordnet ist, erzeugt die Fördereinheit einen Sog, über welchen das Medium durch den Rotationsabscheider gefördert wird. In diesem Fall ist die Fördereinheit eine Saugpumpe. Die Fördereinheit kann selbstansaugend und/oder ventillos ausgebildet sein.

Das Abscheidesystem kann auch zwei Fördereinheiten aufweisen, wobei eine erste Fördereinheit in Strömungsrichtung der Medienströmung vor dem Rotationsabscheider und eine zweite Fördereinheit in Strömungsrichtung der Medienströmung hinter dem Rotationsabscheider angeordnet ist. Die in Strömungsrichtung der Medienströmung vor dem Rotationsabscheider angeordnete Fördereinheit ist beispielsweise eine Kreiselpumpe oder eine Drehkolbenpumpe. Die in Strömungsrichtung der Medienströmung hinter dem Rotationsabscheider angeordnete Fördereinheit ist beispielsweise eine Drehkolbenpumpe. Ein solches Abscheidesystem ist vorzugsweise mit einer Regelung ausgestattet, welche dafür sorgt, dass zulaufseitig, trotz der in Strömungsrichtung der Medienströmung vor dem Rotationsabscheider angeordneten Fördereinheit, ein Gleich- oder Unterdruck vorhanden ist.

Der Rotationsabscheider des Abscheidesystems kann eine eigene Fördereinrichtung aufweisen. Alternativ weist der Rotationsabscheider des Abscheidesystems keine eigene Fördereinrichtung auf.

Die der Erfindung zugrundeliegende Aufgabe wird ferner durch ein Verfahren der eingangs genannten Art gelöst, wobei die Medienströmung bei dem erfindungsgemäßen Verfahren durch einen den Abscheidekanal mit dem Auslassbereich verbindenden Verbindungskanal des zumindest eine Abscheideschaufel umfassenden Rotors von dem Abscheidebereich in den Auslassbereich geleitet wird. Das erfindungsgemäße Verfahren zum Abscheiden von Fremdkörpern aus einer Medienströmung wird insbesondere mittels eines Rotationsabscheiders nach einer der vorstehend beschriebenen Ausführungsformen und/oder mittels eines Abscheidesystems nach einer der vorstehend beschriebenen Ausführungsformen ausgeführt. Hinsichtlich der Vorteile und Modifikationen des erfindungsgemäßen Verfahrens wird somit zunächst auf die Vorteile und Modifikationen des erfindungsgemäßen Rotationsabscheiders und des erfindungsgemäßen Abscheidesystems verwiesen.

Die Medienströmung kann eine Strömung einer Suspension mit beigemengten Störstoffen, wie etwa Sand, Steinen, Glas und/oder Metall, sein. Die Medienströmung kann ferner eine Gülleströmung, eine Sand-Wasser-Kieselgemisch-Strömung oder eine Strömung einer Entsorgungsflüssigkeit sein, welche Feststoffe beinhaltet, etwa zu entsorgende zerkleinerte Essensreste, denen Flaschen- und Besteckteile beigemischt sein können.

In einer bevorzugten Ausführungsform des erfindungsgemäßen Verfahrens wird über den Verbindungskanal durch eine in Strömungsrichtung hinter dem Verbindungskanal angeordnete Fördereinrichtung ein auf die Medienströmung innerhalb des Abscheidebereichs wirkender Sog erzeugt. Vorzugsweise wird innerhalb des Abscheidebereichs des Abscheidergehäuses durch eine Rotationsbewegung der zumindest einen Abscheideschaufel eine Strömung, insbesondere eine Wirbelströmung erzeugt, mittels welcher Fremdkörper durch Fliehkrafteinwirkung radial nach außen gefördert werden.

In einer weiteren bevorzugten Ausführungsform des erfindungsgemäßen Verfahrens wird mittels eines sich zwischen dem Abscheidebereich und dem Auslassbereich befindenden Trennkörpers des Rotationsabscheiders eine Medienströmung zwischen dem Abscheidebereich und dem Auslassbereich jenseits des Verbindungskanals verhindert. Vorzugsweise erfolgt ein Ausleiten von Fremdkörpern aus dem Abscheidebereich oder dem Abscheidergehäuse über einen Fremdkörperauslass des Rotationsabscheiders. Vorzugsweise werden die Fremdkörper in einer durch die zumindest eine Abscheideschaufel verursachten Strömung mittels eines oder mehrerer Leitelemente des Rotationsabscheiders in Richtung des Fremdkörperauslasses geleitet. Nachfolgend werden bevorzugte Ausführungsformen der Erfindung unter Bezugnahme auf die beiliegenden Zeichnungen näher erläutert und beschrieben. Dabei zeigen:
- Fig. 1: ein Ausführungsbeispiel des erfindungsgemäßen Rotationsabscheiders in einer seitlichen Schnittdarstellung;
- Fig. 2: den in der Fig. 1 abgebildeten Rotationsabscheider in einer perspektivischen Darstellung;
- Fig. 3: den in der Fig. 1 abgebildeten Rotationsabscheider in einer perspektivischen Darstellung mit geöffnetem Gehäusedeckel;
- Fig. 4: den in der Fig. 1 abgebildeten Rotationsabscheider in einer Frontansicht mit geöffnetem Gehäusedeckel;
- Fig. 5: das Abscheidergehäuse des in der Fig. 1 abgebildeten Rotationsabscheiders in einer seitlichen Schnittdarstellung;
- Fig. 6: den Rotor samt Antriebseinrichtung des in der Fig. 1 abgebildeten Rotationsabscheiders in einer Seitenansicht;
- Fig. 7: ein weiteres Ausführungsbeispiel des erfindungsgemäßen Rotationsabscheiders in einer seitlichen Schnittdarstellung;
- Fig. 8: den in der Fig. 7 abgebildeten Rotationsabscheider in einer Draufsicht;
- Fig. 9: den in der Fig. 7 abgebildeten Rotationsabscheider in einer perspektivischen Darstellung mit geöffnetem Gehäusedeckel;
- Fig. 10: den in der Fig. 7 abgebildeten Rotationsabscheider in einer Frontansicht mit geöffnetem Gehäusedeckel;
- Fig. 11: das Abscheidergehäuse des in der Fig. 7 abgebildeten Rotationsabscheiders in einer seitlichen Schnittdarstellung;
- Fig. 12: den Rotor samt Antriebseinrichtung des in der Fig. 7 abgebildeten Rotationsabscheiders in einer Seitenansicht;
- Fig. 13: ein weiteres Ausführungsbeispiel des erfindungsgemäßen Rotationsabscheiders in einer perspektiven Darstellung mit geöffnetem Gehäusedeckel;
- Fig. 14: den in der Fig. 13 abgebildeten Rotationsabscheider in einer Explosionsdarstellung;
- Fig. 15: ein weiteres Ausführungsbeispiel des erfindungsgemäßen Rotationsabscheiders in einer perspektiven Darstellung mit geöffnetem Gehäusedeckel;
- Fig. 16: ein weiteres Ausführungsbeispiel des erfindungsgemäßen Rotationsabscheiders in einer perspektiven Darstellung mit geöffnetem Gehäusedeckel;
- Fig. 17: den in der Fig. 16 abgebildeten Rotationsabscheider in einer seitlichen Schnittdarstellung;
- Fig. 18: den in der Fig. 16 abgebildeten Rotationsabscheider in einer Explosionsdarstellung;
- Fig. 19: ein Ausführungsbeispiel des erfindungsgemäßen Abscheidesystems in einer schematischen Darstellung;
- Fig. 20: ein weiteres Ausführungsbeispiel des erfindungsgemäßen Abscheidesystems in einer schematischen Darstellung;
- Fig. 21: ein weiteres Ausführungsbeispiel des erfindungsgemäßen Rotationsabscheiders in einer perspektivischen Darstellung;
- Fig. 22: den in der Fig. 21 abgebildeten Rotationsabscheider in einer seitlichen Schnittdarstellung;
- Fig. 23: den in der Fig. 21 abgebildeten Rotationsabscheider in einer perspektivischen Darstellung mit geöffnetem Gehäusedeckel;
- Fig. 24: den in der Fig. 21 abgebildeten Rotationsabscheider in einer perspektivischen Darstellung mit geöffnetem Gehäusedeckel und ohne Förderschnecke und Abscheideeinrichtung;
- Fig. 25: den Auslassbereich und die Fördereinrichtung des in der Fig. 21 abgebildeten Rotationsabscheiders in einer perspektivischen Darstellung; und
- Fig. 26: den in der Fig. 21 abgebildeten Rotationsabscheider in einer Explosionsdarstellung.

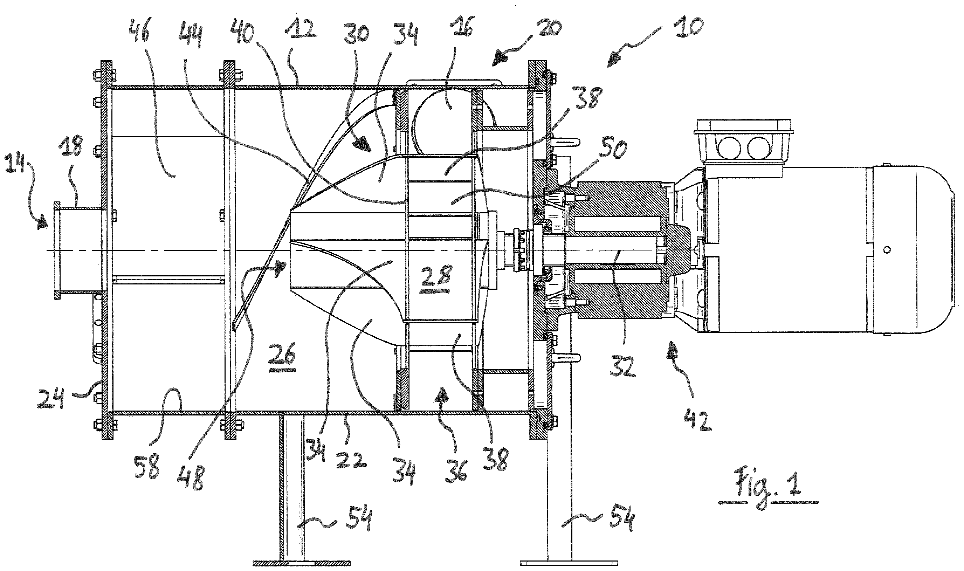

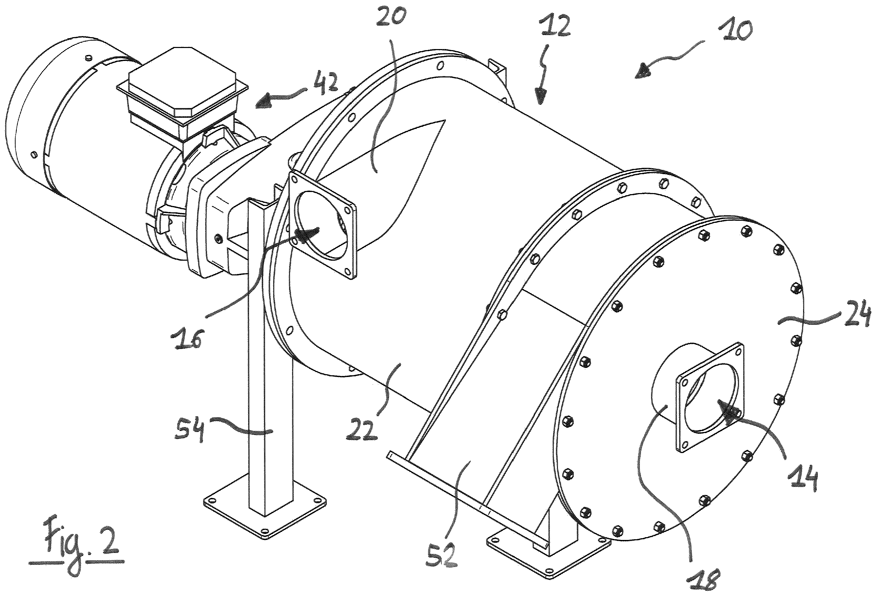

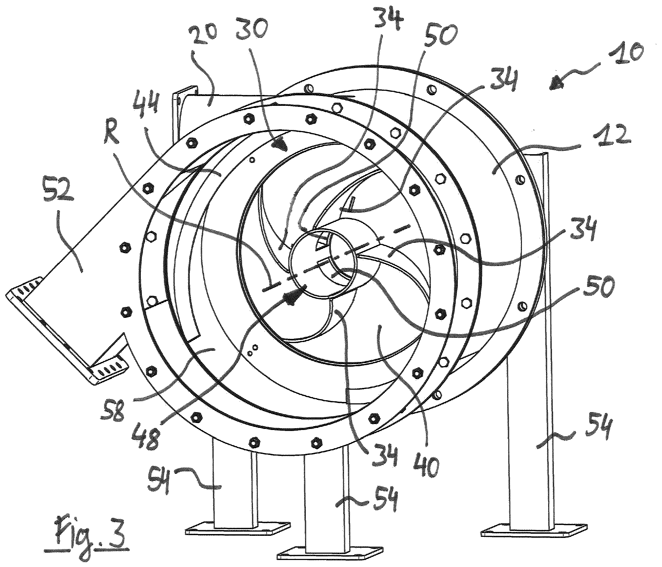

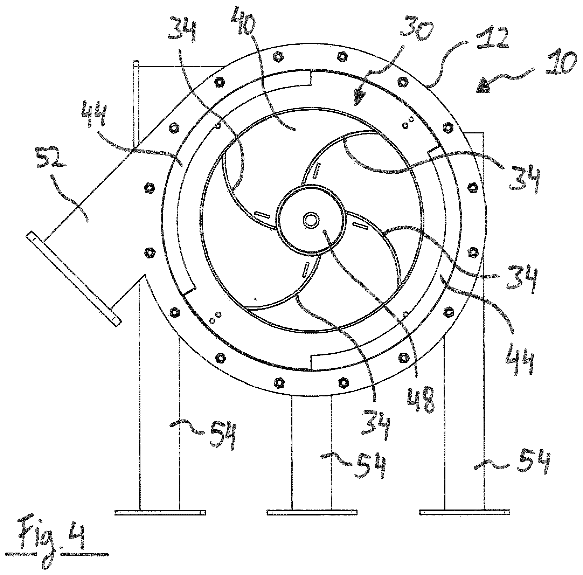

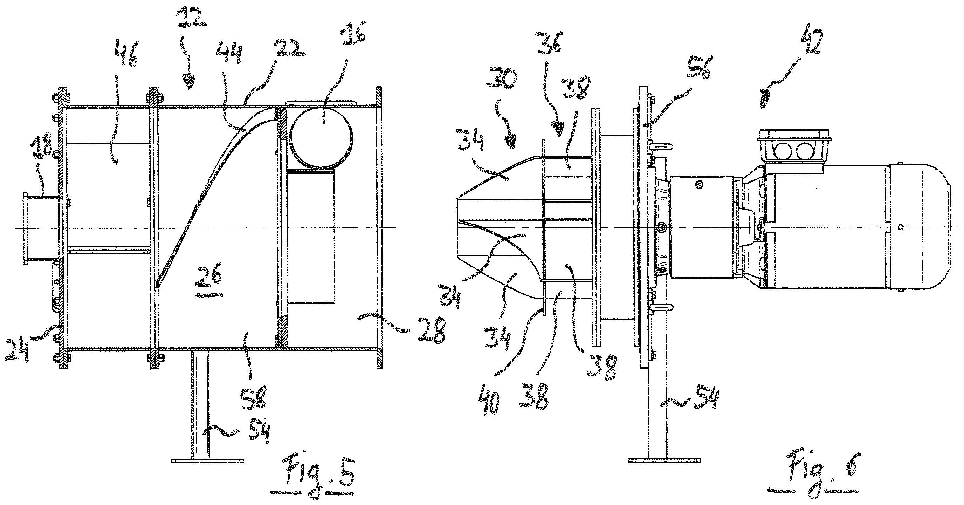

Die Fig. 1 bis 7 zeigen einen Rotationsabscheider 10 zum Abscheiden von Fremdkörpern, beispielsweise Steinen, aus einer Medienströmung, nämlich einer Biogassubstratströmung. Der Rotationsabscheider 10 weist ein im Wesentlichen zylindrisches Abscheidergehäuse 12 auf, in welches über einen Medieneinlass 14 eine Medienströmung zur Fremdkörperabscheidung einleitbar ist. Nach erfolgter Fremdkörperabscheidung kann das Medium über den Medienauslass 16 wieder aus dem Abscheidergehäuse 12 des Rotationsabscheiders 10 ausgeleitet werden.

Der Medieneinlass 14 ist an einer Stirnseite des Abscheidergehäuses 12 angeordnet, wobei die Medienströmung über eine rohrförmige Einlassleitung 18 in das Abscheidergehäuse 12 einleitbar ist.

Der Medienauslass 16 befindet sich in der umlaufenden Mantelfläche des Abscheidergehäuses 12 und ist im oberen Drittel des Abscheidergehäuses 12 angeordnet. Die Auslassleitung 20, über welche die Medienströmung aus dem Abscheidergehäuse 12 ausleitbar ist, erstreckt sich in tangentialer Richtung in Bezug auf den zylindrischen Innenbereich des Abscheidergehäuses 12.

Das Abscheidergehäuse 12 umfasst einen Basiskörper 22 und einen Gehäusedeckel 24. Der Gehäusedeckel 24 ist zerstörungsfrei lösbar mit dem Basiskörper 22 verbunden. Der Medieneinlass 14 bzw. die Einlassleitung 18 sind Bestandteile des Gehäusedeckels 24.

Das Abscheidergehäuse 12 umfasst einen Abscheidebereich 26 und einen Auslassbereich 28. In dem Abscheidebereich 26 erfolgt die Fremdkörperabscheidung aus der Medienströmung. Nach erfolgter Fremdkörperabscheidung wird die Medienströmung aus dem Auslassbereich 28 aus dem Rotationsabscheider 10 ausgeleitet.

Der Rotationsabscheider 10 umfasst einen abschnittsweise in dem Abscheidebereich 26 und abschnittsweise in dem Auslassbereich 28 angeordneten Rotor 30. Der Rotor 30 ist mit einer Antriebswelle 32 verbunden und kann über die Antriebseinrichtung 42 rotierend angetrieben werden. Der Rotor 30 umfasst einen Abscheideschaufelkranz mit mehreren, vorliegend vier, Abscheideschaufeln 34. Ferner umfasst der Rotor 30 vier Förderschaufeln 38 einer Fördereinrichtung 36. Die Abscheideschaufeln 34 sind in dem Abscheidebereich 26 angeordnet. Die Förderschaufeln 38 der Fördereinrichtung 36 sind in dem Auslassbereich 28 angeordnet. Die Abscheideschaufeln 34 und die Förderschaufeln 38 werden durch gemeinsame Schaufelbleche gebildet. Zwischen dem Abscheideschaufelkranz und dem Förderschaufelkranz befindet sich ein Trennkörper 40. Der Trennkörper 40 ist als Platte ausgebildet, welche Bestandteil des Rotors 30 ist.

Der Rotor 30 weist einen den Abscheidebereich 26 mit dem Auslassbereich 28 verbindenden Verbindungskanal 48 auf. Der Verbindungskanal 48 verbindet also den sich auf der Rohseite des Rotationsabscheiders 10 befindenden Abscheidebereich 26 mit dem sich auf der Reinseite des Rotationsabscheiders 10 befindenden Auslassbereich 28. Der Verbindungskanal 48 verläuft koaxial zu der Rotationsachse R des Rotors 30. Der Verbindungskanal wird durch ein zentrales Hohlrohr des Rotors 30 gebildet. Der Verbindungskanal 48 weist auf Höhe der Fördereinrichtung 36 mehrere Durchtrittsöffnungen 50 in der Kanalbewandung auf, über welche die Medienströmung den Förderschaufeln 38 der Fördereinrichtung 36 zugeleitet werden kann. Die Fördereinrichtung 36 ist als Hohlwellenzentrifugalpumpe ausgebildet, wobei der Rotor 30 ein Abscheide- und Förderrotor ist. Die Abscheideschaufeln 34 und die Förderschaufeln 38 sind gekrümmt ausgebildet, wobei die Außenkanten der Abscheideschaufeln 34 auf einer konisch zulaufenden Fläche liegen und die Außenkanten der Förderschaufeln 38 auf einer zylindrischen Fläche liegen. Die in den Abscheidebereich 26 ragenden Außenkanten der Abscheideschaufeln 34 sind nach innen gebogen.

Durch die in Strömungsrichtung hinter dem Verbindungskanal 48 angeordnete Fördereinrichtung 36 kann über den Verbindungskanal 48 eine Saugwirkung in dem Abscheidebereich 26 erzeugt werden. Es entsteht eine sich vor dem Verbindungskanal befindende Ansaugzone, durch welche das Medium von dem Abscheidebereich 26 in den Auslassbereich 28 gesaugt wird. Durch eine Rotationsbewegung der Abscheideschaufeln 34 wird in dem Abscheidebereich 26 eine Wirbelströmung erzeugt, mittels welcher Fremdkörper, beispielsweise Steine, durch Fliehkrafteinwirkung radial nach außen gefördert werden. Die radial nach außen geförderten Fremdkörper werden durch die an der Innenwand 58 des Abscheidergehäuses 12 angeordneten Leitelemente 44 in Richtung eines Fremdkörperauslasses 46 geleitet. Die Leitelemente 44 sind gegenüberliegende Leitstege, welche Schneckenflügel bilden. Über eine Fremdkörperauslassleitung 52, welche sich in tangentialer Richtung in Bezug auf den Abscheidebereich 26 erstreckt, können die abgeschiedenen Fremdkörper einer Entnahmeschleuse des Rotationsabscheiders 10 zugeführt werden. Im Rahmen eines Entleerungsvorgangs können die abgeschiedenen Fremdkörper während des Betriebs des Rotationsabscheiders 10 aus der Entnahmeschleuse entnommen werden.

Der Trennkörper 40 befindet sich zwischen dem Abscheidebereich 26 und dem Auslassbereich 28 und verhindert eine Medienströmung zwischen dem Abscheidebereich 26 und dem Auslassbereich 28 jenseits des Verbindungskanals 48. Auf der der Antriebseinrichtung 42 zugewandten Seite wird der Auslassbereich 28 durch die stirnseitige Rückwand 56 begrenzt. Das Abscheidergehäuse 12 wird über Trägerstreben 54 gegenüber dem Boden abgestützt.

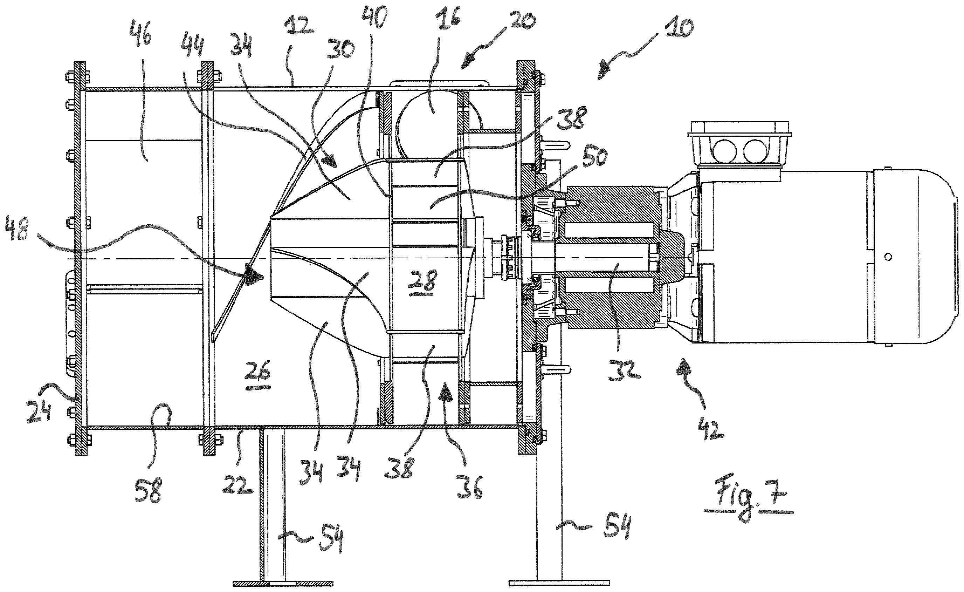

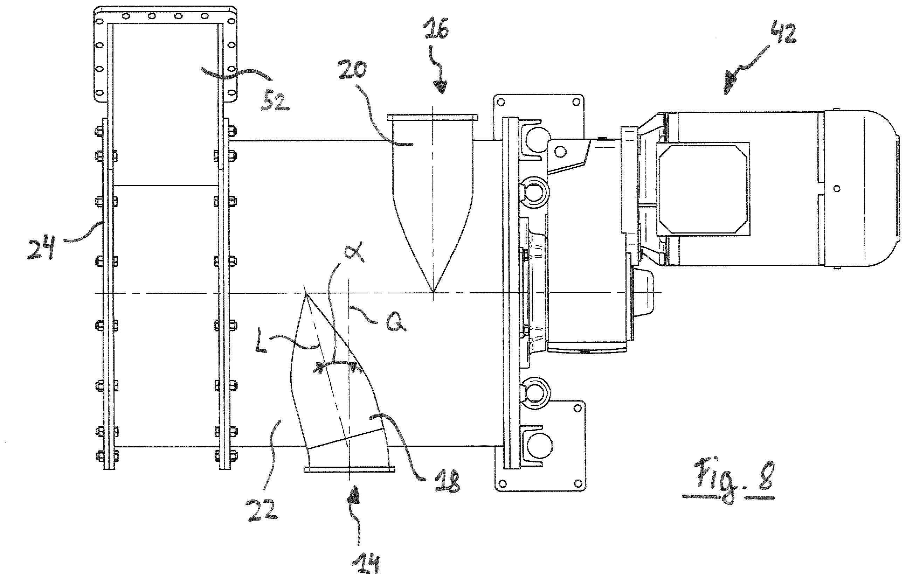

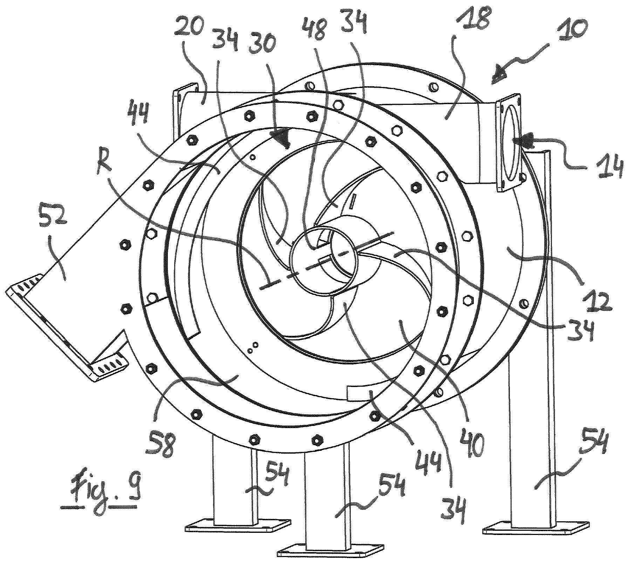

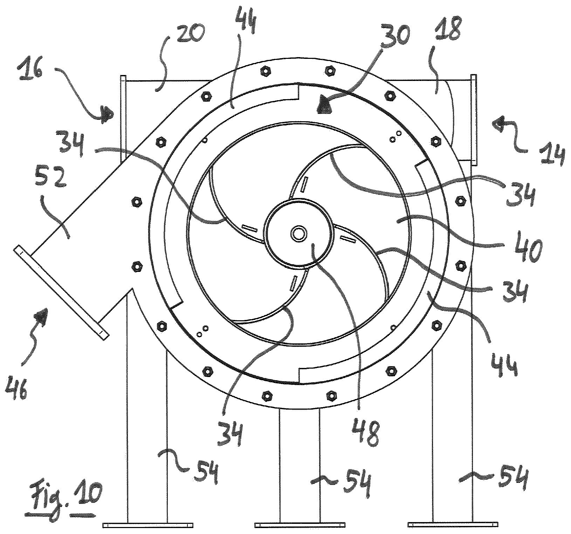

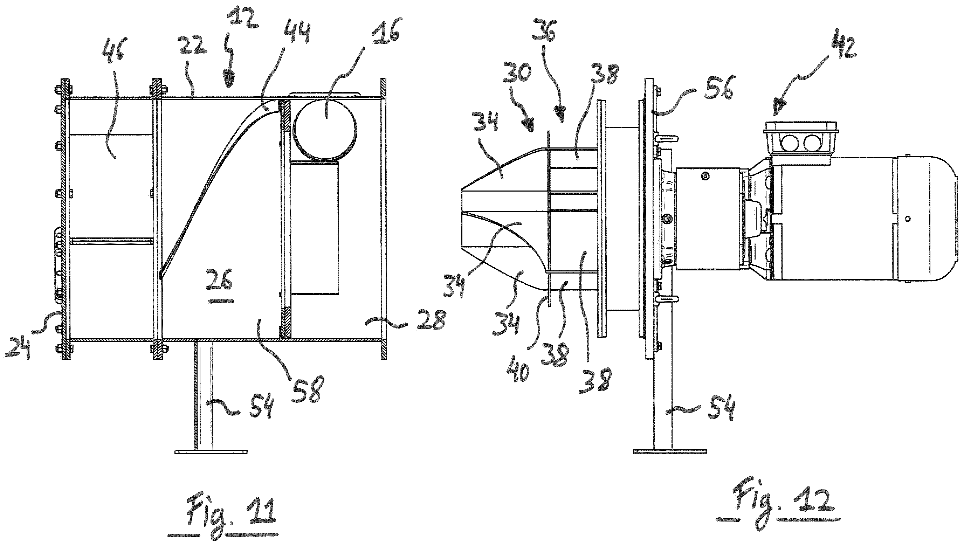

Die Fig. 7 bis 12 zeigen eine alternative Ausführungsform des Rotationsabscheiders 10, bei welcher der Medieneinlass 14 am Außenumfang des Abscheidebereichs 26 angeordnet ist. Die Einlassleitung 18 verläuft schräg gegenüber der Querrichtung, sodass sich ein schräger Eintrittswinkel für die Medienströmung ergibt. Der Schrägstellungswinkel α zwischen der Längsachse L der Einlassleitung 18 und der Querachse Q des Abscheidergehäuses 12 liegt in einem Bereich zwischen 10 Grad und 20 Grad. In anderen Ausführungsformen kann die Längsachse L der Einlassleitung 18 auch parallel zur Querachse Q des Abscheidergehäuses 12 verlaufen. Durch den schrägen Eintrittswinkel der Medienströmung werden die abzuscheidenden Fremdkörper in Richtung des Fremdkörperauslasses 46 gelenkt.

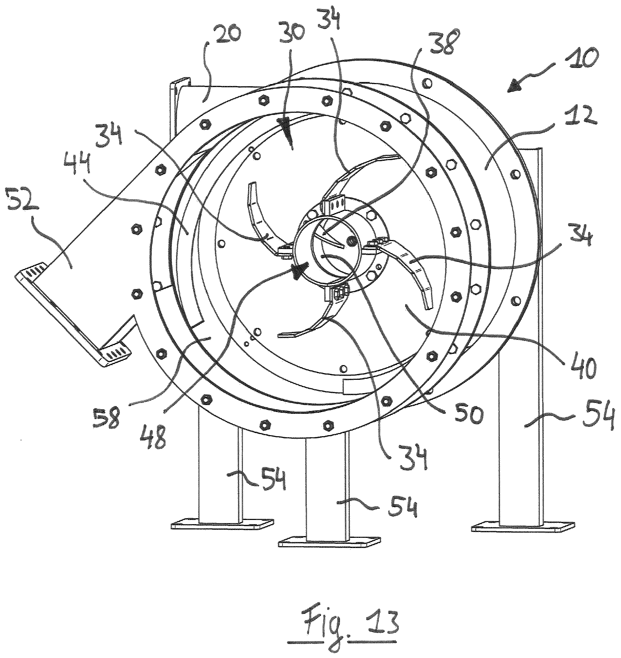

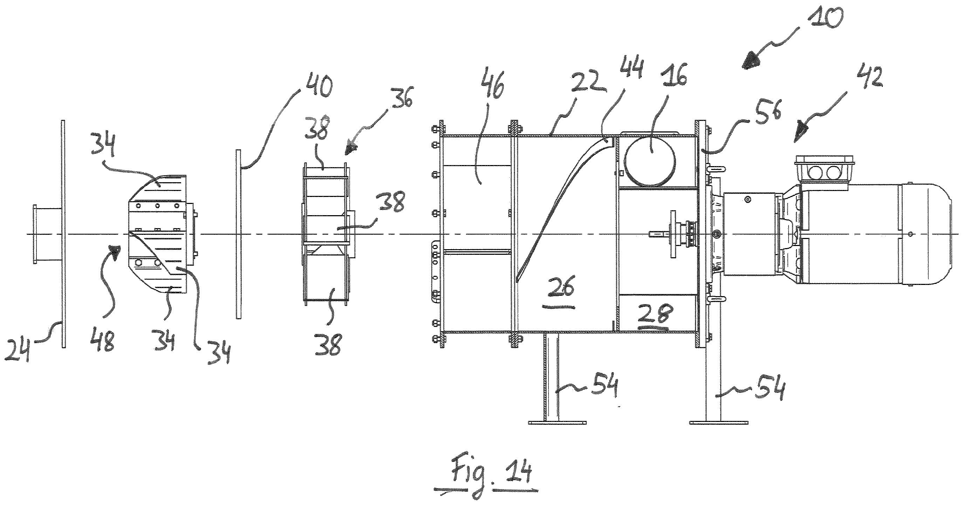

Die Fig. 13 und 14 zeigen einen Rotationsabscheider 10 mit einem feststehenden Trennkörper 40 zwischen dem Abscheidebereich 26 und dem Auslassbereich 28. Der Trennkörper 40 ist als Platte ausgebildet und weist eine zentrale Öffnung auf, durch welche sich der Rotor 30 erstreckt. Der Trennkörper 40 ist an dem Abscheidergehäuse 12 fixiert. Die Förderschaufeln 38 der Fördereinrichtung 36 ragen in den Verbindungskanal 48 hinein.

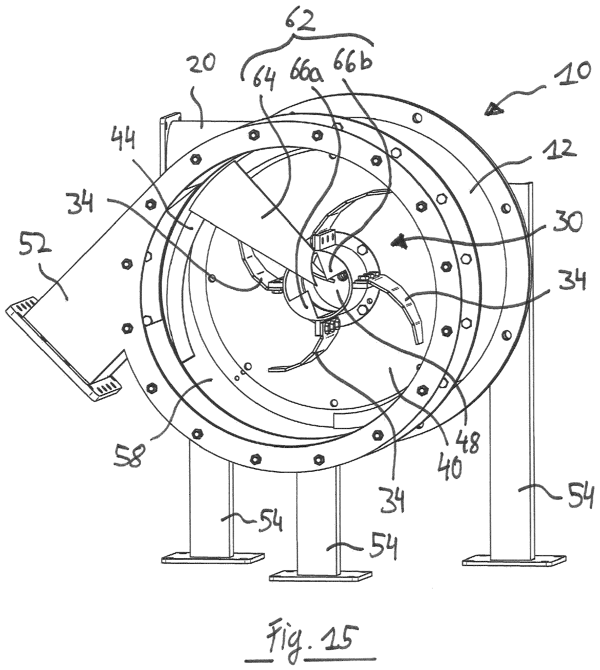

Die Fig. 15 zeigt einen Rotationsabscheider 10 mit einer Schneideinrichtung 62. Die Schneideinrichtung 62 dient zum Zerschneiden von Fasermaterial der Medienströmung vor dem Eintritt in den Verbindungskanal 48. Die Schneideinrichtung 62 umfasst drei als Messer ausgebildete Schneidkörper 64, 66a, 66b. Der Schneidkörper 64 ist feststehend ausgebildet und im Abscheidebereich 26 an dem Abscheidergehäuse 12 befestigt. Die Schneidkörper 66a, 66b sind rotierende Schneidkörper und Bestandteile des Rotors 30. Die Schneidkörper 66a, 66b sind an der Einströmöffnung des Verbindungkanals 48 angeordnet.

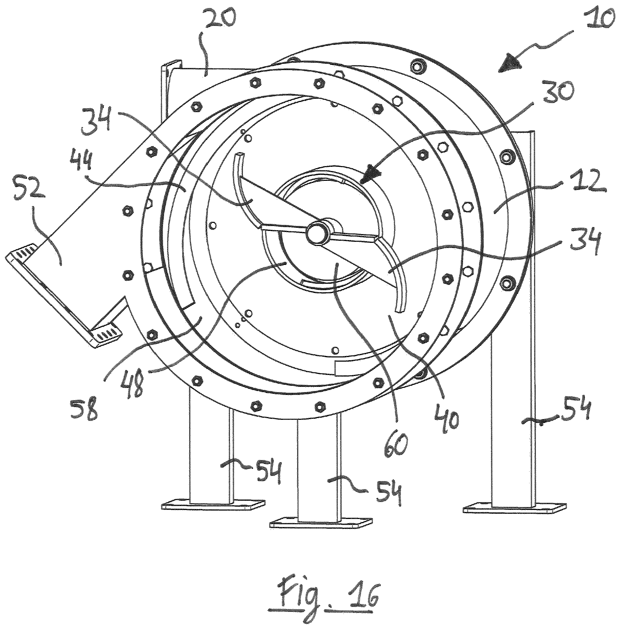

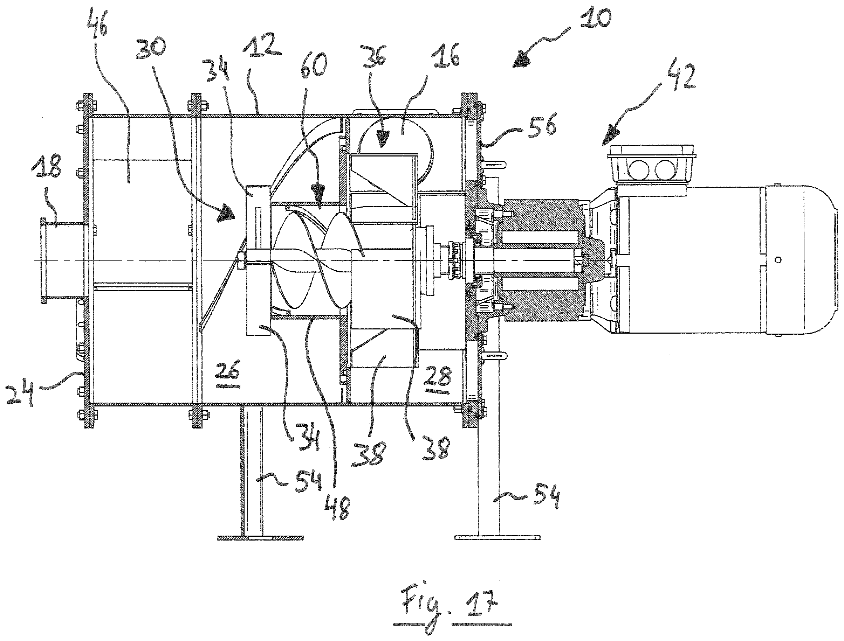

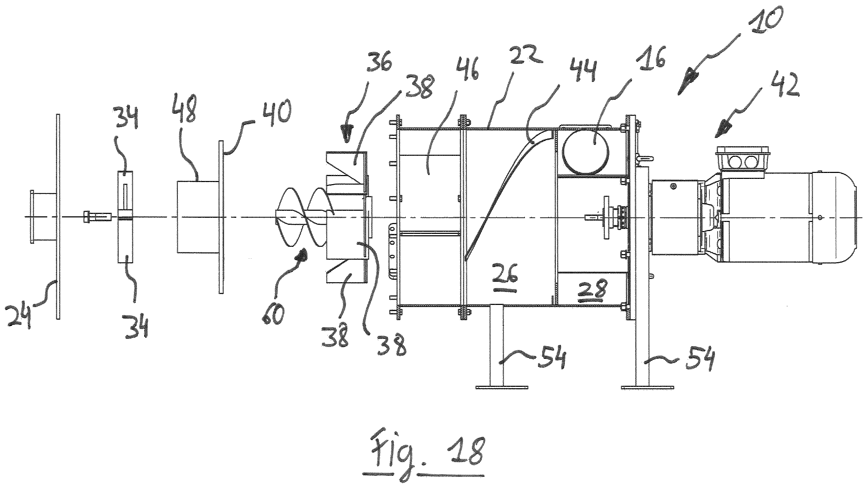

Die Fig. 16 bis 18 zeigen einen Rotationsabscheider 10 mit einem feststehenden Verbindungskanal 48. Durch den Verbindungskanal 48 erstreckt sich der Rotor 30, sodass der Verbindungskanal 48 separat von dem Rotor 30 ausgebildet ist und eine Drehbewegung des Rotors 30 nicht auf den Verbindungskanal 48 übertragen wird. Der Verbindungkanal 48 schließt sich an eine zentrale Durchströmungsöffnung in einer Trennplatte 40 zwischen dem Abscheidebereich 26 und dem Auslassbereich 28 an und ist an der Trennplatte 40 befestigt. In einem sich in dem Verbindungskanal 48 befindenden Abschnitt ist der Rotor 30 als Förderschnecke 60 ausgebildet. Die Förderschnecke 60 unterstützt die Förderung der Medienströmung durch den Verbindungskanal 48.

Die zwei gegenüberliegenden Abscheideschaufeln 34 sind in dem Abscheidebereich 26 und vor dem Verbindungskanal 48 angeordnet.

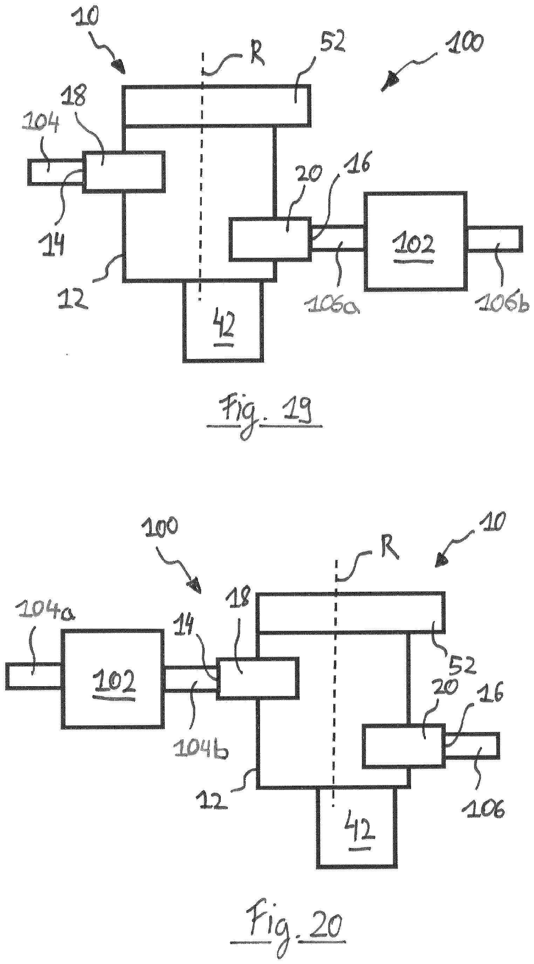

In den in den Fig. 19 und 20 dargestellten Abscheidesystemen 100 wird ein Rotationsabscheider 10 mit einer separaten Fördereinheit 102 kombiniert, welche beabstandet von dem Rotationsabscheider 10 angeordnet ist. Die Fördereinheiten 102 sind jeweils als Verdrängerpumpe, nämlich als Drehkolbenpumpe, ausgebildet.

Die Fig. 19 zeigt ein Abscheidesystem 100, bei welchem dem Rotationsabscheider 10 über eine Zuführleitung 104 eine Fremdkörper beinhaltende Medienströmung zugeführt wird. Nach der Fremdkörperabscheidung wird die Medienströmung über die Abführleitung 106a aus dem Rotationsabscheider 10 herausgeleitet. Die Abführleitung 106a führt zu der Fördereinheit 102. Die Fördereinheit 102 ist in Strömungsrichtung der Medienströmung hinter dem Rotationsabscheider 10 angeordnet. In diesem Fall ist die Fördereinheit 102 eine Saugpumpe. Die Abführleitung 106b schließt sich an die Fördereinheit 102 an.

Die Fig. 20 zeigt ein Abscheidesystem 100, bei welchem die Fördereinheit 102 in Strömungsrichtung der Medienströmung vor dem Rotationsabscheider 10 angeordnet ist. In diesem Fall verursacht die Fördereinheit 102 einen Förderdruck, über welchen das Medium durch den Rotationsabscheider 10 gefördert wird. Die Medienströmung wird über die Zuführleitung 104a der Fördereinheit 102 zugeführt. Die Fördereinheit 102 ist über die Zuführleitung 104b mit dem Rotationsabscheider 10 verbunden. Nach der Fremdkörperabscheidung durch den Rotationsabscheider 10 wird die Medienströmung über Abführleitung 106 aus dem Rotationsabscheider 10 herausgeleitet.

In einer anderen Ausführungsform kann auch eine Fördereinheit 102 in Strömungsrichtung der Medienströmung vor dem Rotationsabscheider 10 und eine weitere Fördereinheit 102 in Strömungsrichtung der Medienströmung hinter dem Rotationsabscheider 10 angeordnet sein.

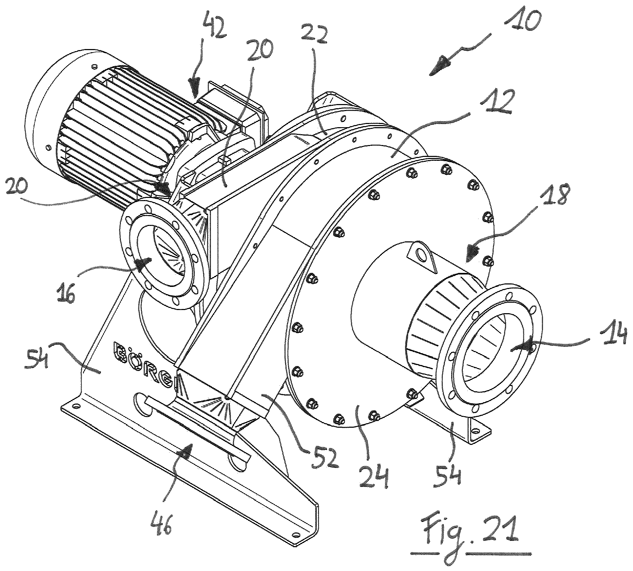

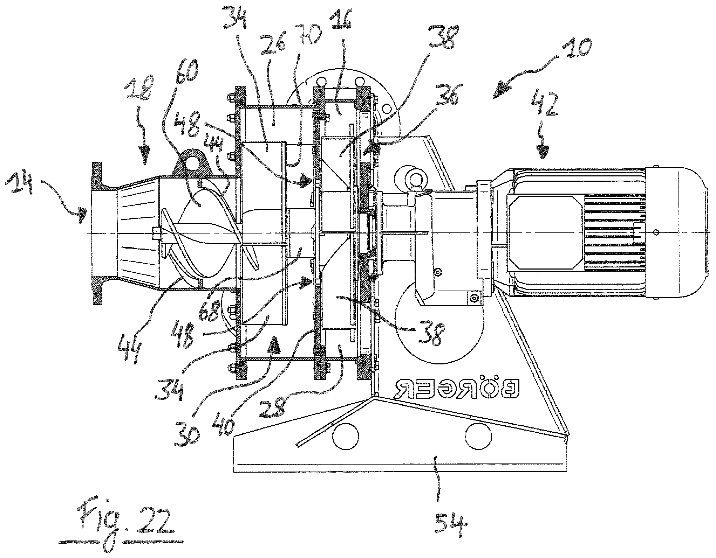

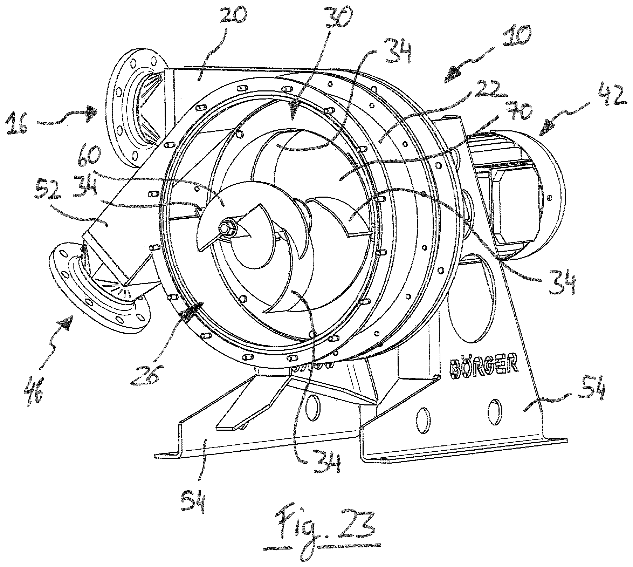

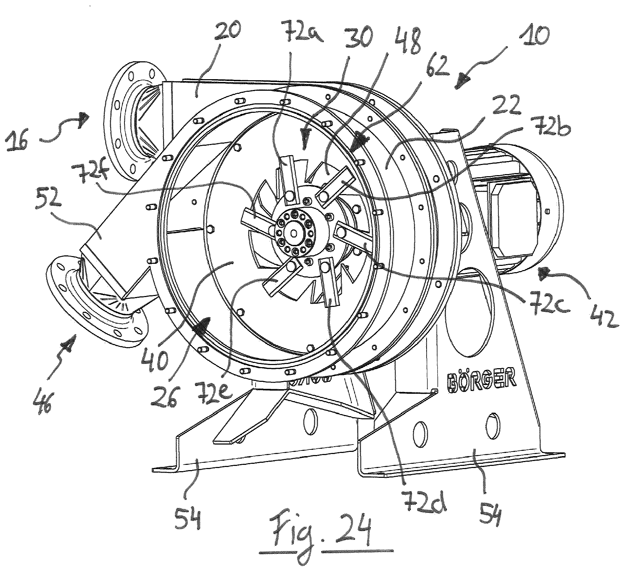

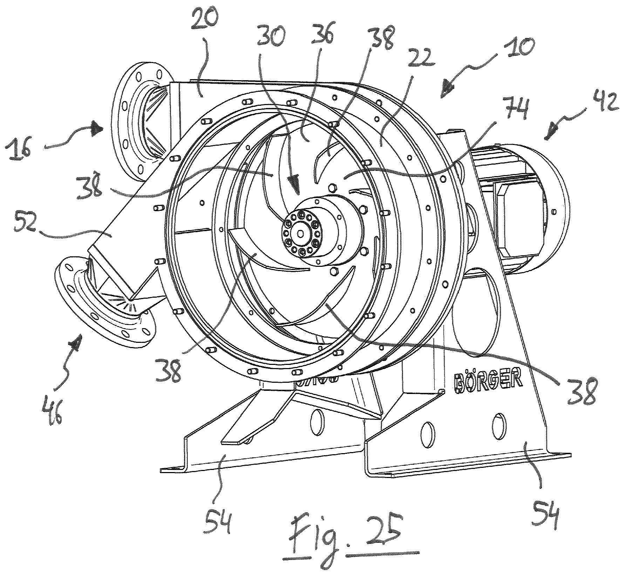

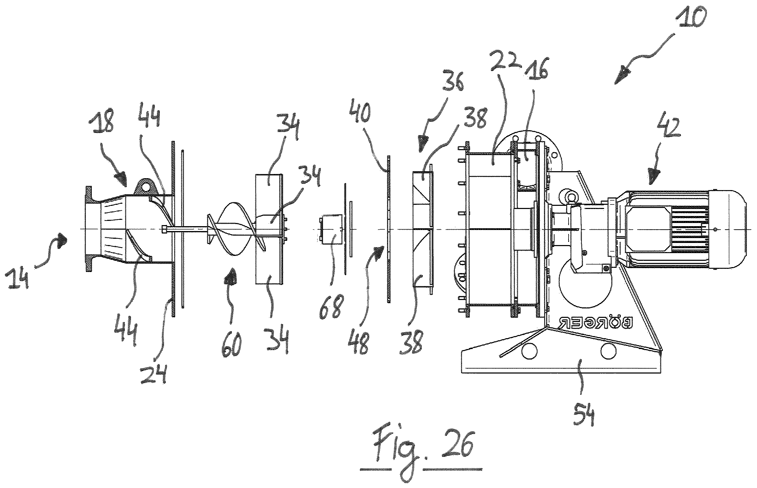

Der in den Fig. 21 bis 26 abgebildete Rotationsabscheider 10 dient ebenfalls zum Abscheiden von Fremdkörpern aus einer Medienströmung. Der Rotationsabscheider 10 umfasst ein Abscheidergehäuse 12, welches einen Abscheidebereich 26 für eine Fremdkörperabscheidung aus der Medienströmung und einen Auslassbereich 28 zum Ausleiten der Medienströmung nach erfolgter Fremdkörperabscheidung aufweist. Ferner befindet sich in dem Abscheidergehäuse 12 ein rotierend angetriebener Rotor 30.

Der Rotor 30 umfasst einen als Förderschnecke 60 ausgebildeten Abschnitt und einen mehrere Abscheideschaufeln 34 aufweisenden Abscheideschaufelkranz. Die Abscheideschaufeln 34 sind mit einer scheibenförmigen Trägerplatte 70 verbunden. Die Trägerplatte 70 und der Abscheideschaufelkranz weisen im Wesentlichen übereinstimmende Durchmesser auf.

Der den als Förderschnecke 60 ausgebildeten Abschnitt des Rotors 30 umgebende Teilbereich des Abscheidergehäuses 12 weist einen kleineren Durchmesser auf als der Abscheideschaufelkranz und der den Abscheideschaufelkranz umgebende Teilbereich des Abscheidergehäuses 12.

Der als Förderschnecke 60 ausgebildete Abschnitt des Rotors 30 verläuft durch eine rohrförmige Einlassleitung 18 des Abscheidergehäuses 12. Die Einlassleitung 18 weist in Strömungsrichtung der Medienströmung vor der Förderschnecke 60 einen Aufweitungsabschnitt auf, in welchem sich der Durchmesser der Einlassleitung 18 in Strömungsrichtung der Medienströmung vergrößert.

Die Förderschnecke 60 fördert die über den Medieneinlass 14 einströmende und Fremdkörper beinhaltende Medienströmung in Richtung des Abscheideschaufelkranzes. An der Innenbewandung des die Förderschnecke 60 umgebenden Teilbereichs des Abscheidergehäuses 12 sind stegförmige Leitelemente 44 angeordnet, welche die gehäuseinterne Strömungsausbildung unterstützen.

Durch die rotatorische Bewegung der Abscheideschaufeln 34 des Rotors 30 werden die sich innerhalb des Abscheidebereichs 26 befindenden Fremdkörper radial nach außen an die den Abscheidebereich 26 umgebende Innenwand des Abscheidergehäuses 12 gefördert und über eine Öffnung in der umlaufenden Mantelfläche des Abscheidergehäuses 12 einer Fremdkörperauslassleitung 52 zugeführt.

Der Fremdkörperauslass 46 kann beispielsweise mit einem Sammelbehältnis, einem Rohrsystem und/oder mit weiteren Aufbereitungs- und/oder Förderaggregaten, wie etwa einer Pumpe, verbunden sein. Vorzugsweise befindet sich in der Fremdkörperauslassleitung 52 oder in Strömungsrichtung der aus der Medienströmung abgeschiedenen Fremdkörper hinter der Fremdkörperauslassleitung 52 ein Absperrschieber oder ein mehrere Absperrschieber umfassendes Schiebersystem, mit welchem eine unbeabsichtigte Medienströmung durch die Fremdkörperauslassleitung 52 vermieden wird.

Der Verbindungskanal 48 zwischen dem Abscheidebereich 26 und dem Auslassbereich 28 ist außerhalb des Rotors 30 ausgebildet und wird durch eine umlaufende Ausnehmung in einem Trennkörper 40 zwischen dem Abscheidebereich 26 und dem Auslassbereich 28 gebildet. Zwischen dem Abscheideschaufelkranz und dem Trennkörper 40 befindet sich eine Abstandshülse 68.

Der Trennkörper 40 ist eine flache Materialscheibe und dient als Gegenschneide für rotierende Schneidkörper 72a-72f einer Schneideinrichtung 62. Die Schneideinrichtung 62 dient zum Zerschneiden von Fasermaterial der Medienströmung vor dem Eintritt in den Verbindungskanal 48 und vor dem Einströmen in den Auslassbereich 28. Die Schneidkörper 72a-72f sind fliegend an dem Rotor 30 gelagert und bilden eine fliegende Messeranordnung.

Der Rotor 30 umfasst ferner eine Fördereinrichtung 36, welche in dem Auslassbereich 28 angeordnet ist und mehrere Förderschaufeln 38 aufweist. Die Förderschaufeln 38 sind mit einer scheibenförmigen Trägerplatte 74 verbunden. Die Trägerplatte 74 und der Förderschaufelkranz weisen im Wesentlichen übereinstimmende Durchmesser auf.

Über die rotierende Fördereinrichtung 36 wird die Medienströmung nach erfolgter Fremdkörperabscheidung im Abscheidebereich 26 der Auslassleitung 20 zugeleitet, um aus dem Rotationsabscheider 10 herausgeleitet zu werden. Die Fördereinrichtung 36 ist als Zentrifugalpumpe ausgebildet und unterstützt die Medienströmung.

### Bezugszeichen

- 10: Rotationsabscheider
- 12: Abscheidergehäuse
- 14: Medieneinlass
- 16: Medienauslass
- 18: Einlassleitung
- 20: Auslassleitung
- 22: Basiskörper
- 24: Gehäusedeckel
- 26: Abscheidebereich
- 28: Auslassbereich
- 30: Rotor
- 32: Antriebswelle
- 34: Abscheideschaufeln
- 36: Fördereinrichtung
- 38: Förderschaufeln
- 40: Trennkörper
- 42: Antriebseinrichtung
- 44: Leitelemente
- 46: Fremdkörperauslass
- 48: Verbindungskanal
- 50: Durchtrittöffnungen
- 52: Fremdkörperauslassleitung
- 54: Trägerstreben
- 56: Rückwand
- 58: Innenwand
- 60: Förderschnecke
- 62: Schneideinrichtung
- 64: Schneidkörper
- 66a, 66b: Schneidkörper
- 68: Abstandshülse
- 70: Trägerplatte
- 72a-72f: Schneidkörper
- 74: Trägerplatte

- 100: Abscheidesystem
- 102: Fördereinrichtung
- 104, 104a, 104b: Zuführleitungen
- 106, 106a, 106b: Abführleitungen

- L: Längsachse
- R: Rotationsachse
- Q: Querachse

- α: Schrägstellungswinkel

## Patentansprüche

1. Rotationsabscheider (10) zum Abscheiden von Fremdkörpern aus einer Medienströmung, insbesondere aus einer Biogassubstratströmung, mit
- einem Abscheidergehäuse (12), welches einen Abscheidebereich (26) für eine Fremdkörperabscheidung aus der Medienströmung und einen Auslassbereich (28) zum Ausleiten der Medienströmung nach erfolgter Fremdkörperabscheidung aufweist; und
- einem zumindest abschnittsweise in dem Abscheidebereich (26) des Abscheidergehäuses (12) angeordneten und rotierend antreibbaren Rotor (30),
wobei der Rotor (30) zumindest eine Abscheideschaufel (34) umfasst und der Abscheidebereich (26) über einen Verbindungskanal (48) mit dem Auslassbereich (28) verbunden ist;
**dadurch gekennzeichnet, dass** der Verbindungskanal (48) außerhalb des Rotors (30) ausgebildet ist.

2. Rotationsabscheider (10) nach Anspruch 1,
**dadurch gekennzeichnet, dass** die zumindest eine Abscheideschaufel (34) als Bestandteil eines, insbesondere um den Verbindungkanal umlaufenden, Abscheideschaufelkranzes ausgebildet ist.

3. Rotationsabscheider (10) nach einem der vorstehenden Ansprüche,
**dadurch gekennzeichnet, dass** der Verbindungskanal (48) koaxial zu der Rotationsachse (R) des Rotors (30) verläuft.

4. Rotationsabscheider (10) nach einem der vorstehenden Ansprüche,
**dadurch gekennzeichnet, dass** der Verbindungskanal (48) durch eine oder mehrere Ausnehmungen in einem Trennkörper (40) zwischen dem Abscheidebereich (26) und dem Auslassbereich (28) gebildet wird.

5. Rotationsabscheider (10) nach einem der vorstehenden Ansprüche,
**dadurch gekennzeichnet, dass** sich zwischen dem Abscheidebereich (26) und dem Auslassbereich (28) ein Trennkörper (40) befindet, welcher dazu eingerichtet ist, eine Medienströmung zwischen dem Abscheidebereich (26) und dem Auslassbereich (28) jenseits des Verbindungskanals (48) im Wesentlichen zu verhindern.

6. Rotationsabscheider (10) nach einem der vorstehenden Ansprüche, **gekennzeichnet durch**
- einen Fremdkörperauslass (46), über welchen Fremdkörper aus dem Abscheidebereich (26) oder dem Abscheidergehäuse (12) ausleitbar sind; und vorzugsweise
- ein oder mehrere Leitelemente (44), welche dazu eingerichtet sind, Fremdkörper in einer durch die zumindest eine Abscheideschaufel (34) verursachten Strömung in Richtung des Fremdkörperauslasses (46) zu leiten.

7. Rotationsabscheider (10) nach einem der vorstehenden Ansprüche, **gekennzeichnet durch** einen Medieneinlass (14), über welchen die Medienströmung in den Abscheidebereich (26) einleitbar ist, wobei der Medieneinlass (14) vorzugsweise an einer dem Rotor (30) gegenüberliegenden Stirnseite des Abscheidebereichs (26) angeordnet ist.

8. Rotationsabscheider (10) nach einem der vorstehenden Ansprüche, **gekennzeichnet durch** eine rotierend antreibbare Fördereinrichtung (36), welche in dem Abscheidergehäuse (12) angeordnet ist und vorzugsweise einen oder mehrere Förderschaufeln (38) aufweist.

9. Rotationsabscheider (10) nach Anspruch 8,
**dadurch gekennzeichnet, dass** die Fördereinrichtung (36) als Bestandteil des Rotors (30) ausgebildet oder drehsteif mit dem Rotor (30) verbunden ist

10. Rotationsabscheider (10) nach Anspruch 8 oder 9, **dadurch gekennzeichnet, dass** sich der Verbindungskanal (48) in die Fördereinrichtung (36) erstreckt und eine oder mehrere Durchtrittöffnungen (50) in der Kanalbewandung aufweist, über welche die Medienströmung der einen oder den mehreren Förderschaufeln (38) der Fördereinrichtung (36) zuleitbar ist.

11. Rotationsabscheider (10) nach einem der vorstehenden Ansprüche,
**dadurch gekennzeichnet, dass** der Verbindungskanal (48) feststehend in dem Abscheidergehäuse (12) angeordnet ist, wobei sich der Rotor (30) vorzugsweise durch den Verbindungskanal (48) erstreckt.

12. Rotationsabscheider (10) nach einem der vorstehenden Ansprüche,
**dadurch gekennzeichnet, dass** der Rotor (30) in einem Abschnitt als Förderschnecke (60) ausgebildet ist.

13. Rotationsabscheider (10) nach einem der vorstehenden Ansprüche,
**gekennzeichnet durch** eine Schneideinrichtung (62), welche dazu eingerichtet ist, Fasermaterial der Medienströmung vor dem Eintritt in den Verbindungskanal (48) oder innerhalb des Verbindungskanals (48) und/oder vor dem Einströmen in den Auslassbereich (28) zu zerschneiden.

14. Abscheidesystem (100), mit
- einer Fördereinrichtung (102) zum Erzeugen oder Unterstützen einer Medienströmung, insbesondere einer Biogassubstratströmung, innerhalb des Abscheidesystems (100), und
- einem von der Fördereinrichtung (102) beabstandeten Rotationsabscheider (10) zum Abscheiden von Fremdkörpern aus der Medienströmung,
**dadurch gekennzeichnet, dass** der Rotationsabscheider (10) nach einem der vorstehenden Ansprüche ausgebildet ist.

15. Verfahren zum Abscheiden von Fremdkörpern aus einer Medienströmung, insbesondere aus einer Biogassubstratströmung, mittels eines Rotationsabscheiders (10), insbesondere mittels eines Rotationsabscheider (10) nach einem der Ansprüche 1 bis 13, oder mittels eines Abscheidesystems nach Anspruch 14, mit den Schritten:
- Einleiten der Medienströmung in einen Abscheidebereich (26) eines Abscheidergehäuses (12) des Rotationsabscheiders (10);
- Abscheiden von Fremdkörpern aus der Medienströmung mittels eines zumindest abschnittsweise in dem Abscheidebereich (26) des Abscheidergehäuses (12) angeordneten, zumindest eine Abscheideschaufel (34) umfassenden und rotierend angetriebenen Rotors (30) des Rotationsabscheiders (10); und
- Ausleiten der Medienströmung nach erfolgter Fremdkörperabscheidung aus einem Auslassbereich (28) des Abscheidergehäuses (12) des Rotationsabscheiders (10);
**dadurch gekennzeichnet, dass** die Medienströmung durch einen den Abscheidebereich (26) mit dem Auslassbereich (28) verbindenden und außerhalb des Rotors (30) ausgebildeten Verbindungskanal (48) von den Abscheidebereich (26) in den Auslassbereich (28) geleitet wird.

## Claims

1. Rotation separator (10) for separation of foreign bodies from a media flow, in particular from a biogas substrate flow, with
- a separator housing (12), which has a separation area (26) for a foreign body separation from the media flow and an outlet area (28) for discharging the media flow after completed foreign body separation; and
- a rotor (30), which is arranged at least in sections in the separation area (26) of the separator housing (12) and is rotatably drivable,
wherein the rotor (30) comprises at least one separator shovel (34) and the separation area (26) is connected via a connection channel (48) with the outlet area (28);
**characterized in that** the connection channel (48) is formed outside the rotor (30).

2. Rotation separator (10) according to claim 1,
**characterized in that** the at least one separator shovel (34) is formed as a component of a separator shovel ring, which is in particular circumferential around the connection channel.

3. Rotation separator (10) according to one of the preceding claims, **characterized in that** the connection channel (48) runs coaxially to the rotation axis (R) of the rotor (30).

4. Rotation separator (10) according to one of the preceding claims, **characterized in that** the connection channel (48) is formed by one or several recesses in a separation body (40) between the separation area (26) and the outlet area (28).

5. Rotation separator (10) according to one of the preceding claims, **characterized in that** between the separation area (26) and the outlet area (28) a separation body (40) is located, which is set up to substantially prevent a media flow between the separation area (26) and the outlet area (28) beyond the connection channel (48).

6. Rotation separator (10) according to one of the preceding claims, **characterized by**
- a foreign body outlet (46), via which foreign bodies are dischargeable from the separation area (26) or the separator housing (12); and preferably
- one or several guide elements (44), which are set up to guide foreign bodies in a flow, caused by the at least one separator shovel (34), in direction of the foreign body outlet (46).

7. Rotation separator (10) according to one of the preceding claims, **characterized by** a media inlet (14), via which the media flow is introduceable into the separation area (26), wherein the media inlet (14) is preferably arranged on a front side of the separation area (26) opposite the rotor (30).

8. Rotation separator (10) according to one of the preceding claims, **characterized by** a rotatably drivable conveyor device (36), which is arranged in the separator housing (12) and preferably has one or several conveyor shovels (38).

9. Rotation separator (10) according to claim 8,
**characterized in that** the conveyor device (36) is formed as a component of the rotor (30) or is connected to the rotor (30) torsionally rigid.

10. Rotation separator (10) according to claim 8 or 9,
**characterized in that** the connection channel (48) extends into the conveyor device (36) and has one or several passage openings (50) in the channel wall, via which the media flow is feedable to the one or the several conveyor shovels (38) of the conveyor device (36).

11. Rotation separator (10) according to one of the preceding claims, **characterized in that** the connection channel (48) is stationary arranged in the separator housing (12), wherein the rotor (30) preferably extends through the connection channel (48).

12. Rotation separator (10) according to one of the preceding claims, **characterized in that** the rotor (30) is formed in a section as a screw conveyor (60).

13. Rotation separator (10) according to one of the preceding claims, **characterized by** a cutting device (62), which is set up to cut fiber material of the media flow before entering the connection channel (48) or within the connection channel (48) and/or before flowing into the outlet area (28).

14. Separation system (100), with
- a conveyor device (102) for generating or supporting a media flow, in particular a biogas substrate flow, within the separation system (100), and
- a rotation separator (10) spaced from the conveyor device (102) for separating foreign bodies from the media flow,
**characterized in that** the rotation separator (10) is formed according to one of the preceding claims.

15. Process for separating foreign bodies from a media flow, in particular from a biogas substrate flow, by means of a rotation separator (10), in particular by means of a rotation separator (10) according to one of the claims 1 to 13, or by means of a separation system according to claim 14, with the steps:
- Introducing the media flow in a separation area (26) of a separator housing (12) of the rotation separator (10);
- Separating from foreign bodies from the media flow by means of a rotor (30) of the rotation separator (10), which is arranged at least in sections in the separation area (26) of the separator housing (12), comprises at least one separator shovel (34) and is rotatably driven; and
- Discharging the media flow after completed foreign body separation from an outlet area (28) of the separator housing (12) of the rotation separator (10);
**characterized in that** the media flow is guided from the separation area (26) into the outlet area (28) through a connection channel (48), which connects the separation area (26) to the outlet area (28) and is formed outside the rotor (30).

## Revendications

1. Séparateur rotatif (10) pour séparer des corps étrangers d'un flux de fluides, notamment d'un flux de substrat de biogaz, avec
- un boîtier de séparateur (12) qui présente une zone de séparation (26) pour une séparation de corps étrangers du flux de fluides et une zone d'écoulement (28) pour évacuer le flux de fluides après la séparation de corps étrangers ; et
- un rotor (30) disposé au moins partiellement dans la zone de séparation (26) du boîtier de séparateur (12) et pouvant être entraîné en rotation,
ledit rotor (30) comprenant au moins une aube de séparation (34), et la zone de séparation (26) étant reliée à la zone d'écoulement (28) par un canal de liaison (48) ;
**caractérisé en ce que** le canal de liaison (48) est conçu à l'extérieur du rotor (30).

2. Séparateur rotatif (10) selon la revendication 1,
**caractérisé en ce que** ladite au moins une aube de séparation (34) est conçue en tant que partie intégrante d'une couronne d'aubes de séparation, entourant notamment le canal de liaison.

3. Séparateur rotatif (10) selon l'une quelconque des revendications précédentes,
**caractérisé en ce que** le canal de liaison (48) est coaxial à l'axe de rotation (R) du rotor (30).

4. Séparateur rotatif (10) selon l'une quelconque des revendications précédentes,
**caractérisé en ce que** le canal de liaison (48) est formé par un ou plusieurs évidements dans un corps de séparation (40) entre la zone de séparation (26) et la zone d'écoulement (28).

5. Séparateur rotatif (10) selon l'une quelconque des revendications précédentes,
**caractérisé en ce qu'**un corps de séparation (40) se trouve entre la zone de séparation (26) et la zone d'écoulement (28), lequel corps de séparation est susceptible d'empêcher sensiblement un flux de fluides entre la zone de séparation (26) et la zone d'écoulement (28) au-delà du canal de liaison (48).

6. Séparateur rotatif (10) selon l'une quelconque des revendications précédentes,
**caractérisé par**
- une bouche d'écoulement de corps étrangers (46) par laquelle des corps étrangers peuvent être évacués de la zone de séparation (26) ou du boîtier de séparation (12) ; et de préférence
- un ou plusieurs éléments de guidage (44) qui sont susceptibles de guider des corps étrangers dans un flux provoqué par ladite au moins une aube de séparation (34) en direction de la bouche d'écoulement de corps étrangers (46).

7. Séparateur rotatif (10) selon l'une quelconque des revendications précédentes,
**caractérisé par** une bouche d'entrée de fluides (14) par laquelle le flux de fluides peut être introduit dans la zone de séparation (26), ladite bouche d'entrée de fluide (14) étant agencée de préférence sur une face frontale de la zone de séparation (26) opposée au rotor (30).

8. Séparateur rotatif (10) selon l'une quelconque des revendications précédentes,
**caractérisé par** un dispositif de convoyage (36) pouvant être entraîné en rotation, qui est disposé dans le boîtier de séparateur (12) et qui présente de préférence une ou plusieurs pales de convoyage (38).

9. Séparateur rotatif (10) selon la revendication 8,
**caractérisé en ce que** ledit dispositif de convoyage (36) est réalisé en tant que partie intégrante du rotor (30) ou est relié au rotor (30) de manière rigide en rotation.

10. Séparateur rotatif (10) selon la revendication 8 ou 9,
**caractérisé en ce que** le canal de liaison (48) s'étend dans le sens de convoyage (36) et présente un ou plusieurs orifices de passage (50) dans la paroi du canal, par lesquels le flux de fluide peut être introduit vers l'une ou les plusieurs pales de convoyage (38) du dispositif de convoyage (36).

11. Séparateur rotatif (10) selon l'une quelconque des revendications précédentes,
**caractérisé en ce que** le canal de liaison (48) est agencé de manière fixe dans le boîtier de séparateur (12), le rotor (30) s'étendant de préférence au travers du canal de liaison (48).

12. Séparateur rotatif (10) selon l'une quelconque des revendications précédentes,
**caractérisé en ce que** le rotor (30) est conçu sous forme de vis sans fin (60) dans une section.

13. Séparateur rotatif (10) selon l'une quelconque des revendications précédentes,
**caractérisé par** un dispositif de coupe (62) qui est susceptible de découper la matière fibreuse du flux de fluides avant la bouche d'entrée dans le canal de liaison (48) ou à l'intérieur du canal de liaison (48) et/ou avant qu'il ne pénètre dans la zone d'écoulement (28).

14. Système séparateur (100), avec
- un dispositif de convoyage (102) pour générer ou soutenir un flux de fluides, notamment un flux de substrat de biogaz à l'intérieur du système de séparation (100), et
- un séparateur rotatif (102) espacé du dispositif de convoyage (10) pour séparer les corps étrangers du flux de fluides,
**caractérisé en ce que** le séparateur rotatif (10) est conçu selon l'une quelconque des revendications précédentes

15. Procédé de séparation de corps étrangers d'un flux de fluides, notamment d'un flux de substrats de biogaz, au moyen d'un séparateur rotatif (10), notamment au moins d'un séparateur rotatif (10) selon l'une quelconque des revendications 1 à 13 ou au moyen d'un système de séparation selon la revendication 14, comprenant les étapes :
- introduction du flux de fluides dans une zone de séparation (26) d'un boîtier de séparateur (12) du séparateur rotatif (10) ;
- séparation de corps étrangers du flux de fluides au moyen d'un rotor (30) du séparateur rotatif (10) agencé au moins partiellement dans la zone de séparation (26) du boîtier de séparateur (12), comprenant au moins une aube de séparation (34) et entraîné en rotation ; et
- évacuation du flux de fluides, après que la séparation de corps étrangers aura été exécutée, d'une zone d'écoulement (28) du boîtier de séparateur (12) du séparateur rotatif (10) ;
**caractérisé en ce que** le flux de fluides est entraîné de la zone de séparation (26) dans la zone d'écoulement (28) par un canal de liaison (48) reliant la zone de séparation (26) à la zone d'écoulement (28) et conçu à l'extérieur du rotor (30).
